(19) Europäisches Patentamt
European Patent Office
Office européen des brevets

(11) Veröffentlichungsnummer : **0 131 859**
**B1**

(12) # EUROPÄISCHE PATENTSCHRIFT

(45) Veröffentlichungstag der Patentschrift : **04.03.87**

(51) Int. Cl.[4] : **C 07 C118/00**, C 07 C119/042, C 07 D295/12, C 07 D265/28

(21) Anmeldenummer : **84107872.8**

(22) Anmeldetag : **05.07.84**

(54) **Verfahren zur Herstellung von in 3-Stellung heterocyclisch oder durch Dialkylamino substituierten 2,2-Dialkylpropylisocyanaten.**

(30) Priorität : **13.07.83 DE 3325185**

(43) Veröffentlichungstag der Anmeldung : **23.01.85 Patentblatt 85/04**

(45) Bekanntmachung des Hinweises auf die Patenterteilung : **04.03.87 Patentblatt 87/10**

(84) Benannte Vertragsstaaten : **BE CH DE FR GB LI**

(56) Entgegenhaltungen :
**EP-A- 0 002 708**
**EP-A- 0 052 254**
**EP-A- 0 061 013**
**DE-A- 2 325 826**
**DE-C- 1 171 421**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Towae, Friedrich, Dr.**
**Schwedlerstrasse 118**
**D-6700 Ludwigshafen (DE)**

**EP 0 131 859 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von in 3-Stellung heterocyclischen oder 3-Dialkylamino-2,2-dialkyl-propyl-isocyanaten durch thermische Spaltung von in 3-Stellung heterocyclischen oder N-(3-Dialkylamino-2,2-dialkyl-propyl)-carbamidsäureestern bei einer Temperatur von mehr als 300 °C und Abkühlung des Reaktionsgemisches auf weniger als 60 °C.

Isocyanate werden im allgemeinen durch Phosgenierung von primären Aminen hergestellt (Houben-Weyl, Methoden der Organischen Chemie, Band VIII, Seiten 119 bis 121), jedoch besitzt dieses Verfahren wegen der Toxizität des Phosgens sowie der Korrosivität des HCl und des damit verbundenen hohen technologischen Aufwands schwerwiegende Nachteile. So entstehen hohe Investitionskosten, speziell bei der Herstellung geringer Mengen an Isocyanaten.

Es ist bekannt, Isocyanate aus Carbamidsäurechloriden in Gegenwart von organischen Basen wie tertiären Aminen oder N,N-Dialkylcarbonsäureamiden (DE-OS 1 593 554) in organischen Lösungsmitteln herzustellen. Auch mit wäßrigen Lösungen oder Suspensionen von anorganischen Basen, wie Alkalihydroxiden, Erdalkalihydroxiden, Alkalicarbonaten, Erdalkalicarbonaten oder Alkalihydrogencarbonaten, können Isocyanate erhalten werden (GP-PS 1 208 862). US-Patentschrift 3 465 023 weist ausdrücklich darauf hin, daß die Bildung von Chlorwasserstoff bei der Isocyanatherstellung die Reaktionsfreudigkeit der Endstoffe herabsetzt und daher die Bindung der Säure bei dem Verfahren wichtig ist. Auch ergeben sich Schwierigkeiten bei der Destillation des Isocyanats und Korrosion in den Anlagen. Die genannten Verfahren haben den Nachteil, daß die Isocyanate in einem Medium entstehen, in dem sie gegen Zersetzung empfindlich sind. So ist es aus Houben-Weyl, Methoden der Organischen Chemie, Band VIII, Seite 136 (1952) bekannt, daß Isocyanate in Gegenwart tertiärer Amine dimerisieren. Gegenüber wäßrigem Alkali sind sie äußerst instabil und gehen selbst bei Verwendung stöchiometrischer Mengen an wäßrigem Alkali zu einem großen Teil über Carbamidsäuren in Carbaminate über.

Ein weiterer Weg zu Isocyanaten besteht in der thermischen Spaltung von Urethanen (DE-OS 2 635 490, DE-OS 2 410 505), die aus Nitroverbindungen, Kohlenmonoxid und Alkohol erhalten werden (DE-OS 2 623 694, EP-Anm. 0 000 563, DE-OS 2 614 101). Dieses Verfahren wurde jedoch bislang nur für aromatische Isocyanate angewandt.

Die Herstellung organischer Isocyanate durch thermische Spaltung von N-Alkyl- bzw. N-Arylthiocarbamidsäure-S-alkylestern, Abschrecken der Spaltprodukte und Abtrennung der flüssigen von der gasförmigen Phase ist in der DE-AS 1 171 421 beschrieben. Während dieses Verfahren im Fall von alkoxysubstituierten Alkylisocyanaten noch schlechte bis mäßige Ausbeuten von ca. 30 bis 70 % liefert, wurden, wie aus den Beispielen 8 und 9 hervorgeht, bei der Herstellung von N,N-dialkylaminosubstituierten Alkylisocyanaten lediglich Ausbeuten von 20 bis 24 % erhalten.

Es ist aus der DE-OS 3 021 299 bekannt, daß man aliphatische Isocyanate durch Spaltung von Oxalsäureesteramiden bei einer Temperatur von mindestens 300 °C herstellen kann. Als in Frage kommende Ausgangsstoffe (Seite 5, Zeile 24, bis Seite 6, Zeile 10) werden unter den am Stickstoff- und Sauerstoffatom durch aliphatische Gruppen substituierten Oxalsäureesteramiden nur solche beschrieben, die keine tert. Aminfunktionen tragen. Die Beispiele (7 und 8) zeigen lediglich Oxalsäure-n-decylester-N-methylamid und das Oxalsäuremethylester-n-octylamid als aliphatische Ausgangsstoffe, die übrigen Beispiele verwenden N-Phenylamide von Oxalsäuremethylester und -butylester.

Zur Herstellung von Aminoalkylgruppen enthaltenden Isocyanaten sind alle genannten Herstellmethoden jedoch ungeeignet, da durch die Amingruppe der bei der Spaltung der Carbamidsäurechloride freigesetzte Chlorwasserstoff gebunden wird, wodurch es praktisch unmöglich wird, die freien Isocyanate in akzeptablen Ausbeuten zu erhalten. Bei der thermischen Spaltung Amingruppen enthaltender Urethane waren schlechte Isocyanatausbeuten zu erwarten, da Isocyanate in Gegenwart tert. Amine sehr leicht zu Dimeren oder Trimeren reagieren (DE-OS 2 325 826).

Verwendet man bei den in der DE-OS 3 021 299 beschriebenen Verfahren Oxalsäureester-N-aminoalkylamide, die in 2-Stellung an der Alkylgruppe 2 Alkylreste besitzen, so erhält man in erheblichen Mengen Crackprodukte (Vergleichsbeispiel 7).

Es wurde nun gefunden, daß man in 3-Stellung heterocyclische oder 3-Dialkylamino-2,2-dialkylpropylisocyanate der Formel

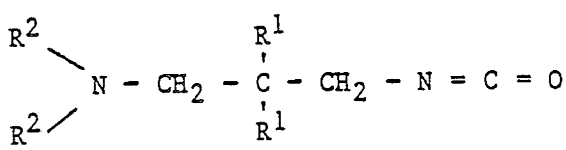

(I)

worin die einzelnen Reste $R^1$ und $R^2$ gleich oder verschieden sein können und jeweils einen aliphatischen Rest bedeuten, die Reste $R^2$ auch zusammen mit dem benachbarten Stickstoffatom Glieder eines heterocyclischen Restes bezeichnen, durch thermische Spaltung von Carbamidsäureestern vorteilhaft erhält, wenn man in 3-Stellung heterocyclische oder N-(3-Dialkylamino-2,2-dialkylpropyl)-carbamidsäureester der Formel

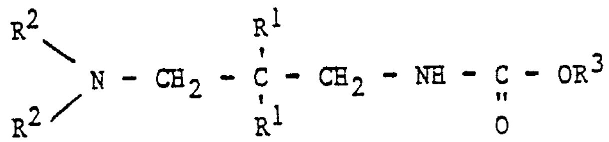

(II)

worin $R^1$ und $R^2$ die vorgenannte Bedeutung besitzen und $R^3$ einen aliphatischen, cycloaliphatischen, araliphatischen oder aromatischen Rest bezeichnet, in einer Inertgasphase bei einer Temperatur von mehr als 300 °C spaltet und das so gebildete Reaktionsgemisch auf weniger als 60 °C abkühlt.

Es wurde weiterhin gefunden, daß man dass Verfahren vorteilhaft ausführt, wenn man die Spaltung in einer Stickstoffphase in Gegenwart von gasdurchlässigen, metallischen, durch Halogenwasserstoff aktivierten Füllkörpern durchführt.

Die Umsetzung kann für den Fall der Verwendung von N-(3-Dimethylamino-2,2-dimethylpropyl)-carbamidsäuremethylester durch die folgenden Formeln wiedergegeben werden :

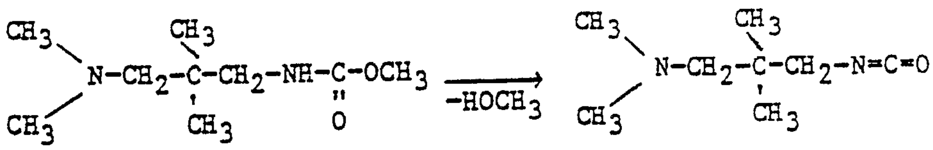


Im Hinblick auf die bekannten Verfahren liefert das Verfahren nach der Erfindung auf einfachem und wirtschaftlicherem Wege die neuen in 3-Stellung heterocyclischen oder 3-Dialkylamino-2,2-dialkylpropyldiisocyanate in guter Ausbeute und Reinheit. Teure Lösungsmittel und zusätzliche Basen werden nicht benötigt. Diese vorteilhaften Ergebnisse sind überraschend, denn nach dem Stand der Technik war insbesondere die Bildung von Polymeren bzw. Zersetzungsprodukten unter den erfindungsgemäßen hohen Temperaturen zu erwarten. Es war im Hinblick auf die DE-OS 2 325 826 unerwartet, daß Isocyanurate nicht in deutlichem Maße gebildet werden.

Die als Ausgangsstoffe II eingesetzten in 3-Stellung heterocyclischen oder N-(3-Dialkylamino-2,2-dialkylpropyl)-carbamidsäureester können beispielsweise durch die Umsetzung von entsprechenden Aminen mit Chlorkohlensäureester (Houben-Weyl, loc. cit., Seite 137 bis 199) oder mit Alkoholen und Harnstoff (EP-PS 0 018 586) hergestellt werden.

Bevorzugte Ausgangsstoffe II und dementsprechend bevorzugte Endstoffe I sind solche, in deren Formeln die einzelnen Reste $R^1$, $R^2$ und $R^3$ gleich oder verschieden sein können und jeweils einen Alkylrest mit 1 bis 18, vorzugsweise 1 bis 8, insbesondere 1 bis 6 Kohlenstoffatomen, vorteilhaft die Methyl-, Ethyl-, Propyl-, Isopropyl-, Butyl-, Isobutyl-, sek-Butyl- oder Pentylgruppe, einen Alkenylrest mit 2 bis 12, zweckmäßig 2 bis 6 Kohlenstoffatomen, bedeuten, $R^3$ auch für einen Cycloalkylrest mit 3 bis 8 Kohlenstoffatomen, einen Alkylarylrest oder einen Aralkylrest mit 7 bis 12 Kohlenstoffatomen oder einen Phenylrest steht, die beiden Reste $R^2$ auch zusammen mit dem benachbarten Stickstoffatom einen heterocyclischen 3- bis 7-, zweckmäßig 5- bis 6-gliedrigen Ring, der noch ein weiteres Stickstoffatom oder ein Sauerstoffatom oder ein Schwefelatom enthalten kann, bezeichnen können. Die vorgenannten Reste können noch durch unter den Reaktionsbedingungen inerte Gruppen, z. B. Alkylgruppen mit jeweils 1 bis 4 Kohlenstoffatomen, substituiert sein.

Vorteilhaft werden Ausgangsstoffe II verwendet, bei denen die Siedepunkte der Spaltprodukte Isocyanat und Alkohol so weit auseinanderliegen, daß durch fraktionierende Kondensation eine möglichst quantitative Trennung der beiden Endprodukte erreicht wird. Diese Eigenschaft besitzen beispielsweise Carbamidsäureester, in denen $R^3$ für den Methyl-, Ethyl-, Propyl-, Butyl-, Isobutyl-, 1- oder 2-Naphthylrest steht.

Es kommen beispielsweise als Ausgangsstoffe II in Frage :

2,2-Dimethyl-3-di-hexyl-, 2,2-Dimethyl-3-di-methyl-, 2,2-Dimethyl-3-di-isopropyl-, 2,2-Dimethyl-3-diethyl-, 2,2-Dimethyl-3-di-sek.-butyl-, 2,2-Dimethyl-3-di-propyl-, 2,2-Dimethyl-3-di-pentyl-, 2,2-Dimethyl-3-di-isopentyl-, 2,2-Dimethyl-3-di-(2'-ethylhexyl)-, 2,2-Dimethyl-3-di-nonyl-, 2,2-Dimethyl-3-di-decyl-, 2,2-Dimethyl-3-di-n-octyl-, 2,2-Dimethyl-3-di-heptyl-, 2,2-Dimethyl-3-di-isobutyl-, 2,2-Dimethyl-3-di-n-butyl-aminopropyl-(N)-carbamidsäuremethylester, N'-Methyl-N'-ethyl-2,2-dimethyl-3-aminopropyl-(N)-carbamidsäuremethylester ; 3-Piperidino-, 3-Morpholino-, 3-Pyrrolidino-, 3-Aziridino-, 3-Thiomorpholino-2,2-dimethyl-propyl-(N)-carbamidsäuremethylester ; entsprechende in 2,2-Stellung gleich oder unterschiedlich zweifach durch Ethyl-, n-Propyl-, iso-Propyl-, n-Butyl-, iso-Butyl-, sek.-Butyl-, 2-Methylbutyl-(1)-, 3-Methylbutyl-(1)-, 2-Methylbutyl-(2)-, 3-Methylbutyl-(2)-, Pentyl-(1)-, Pentyl-(2)-, Pentyl-(3)-gruppen substituierte Methylester vorgenannter Propyl-(N)-carbamidsäuren ; homologe Ethyl-, n-Propyl-, iso-Propyl-, n-Butyl-, iso-Butyl-, sek.-Butyl-, Pentyl-(1)-, Decyl-, Benzyl-, Phenyl-, 1- bzw. 2-Naphthyl-, Tolylester vorgenannter Propyl-(N)-carbamidsäuren.

Die Umsetzung wird vorteilhaft bei einer Temperatur von mehr als 300 bis 600 °C, vorzugsweise von 301 bis 550 °C, insbesondere 400 bis 535 °C drucklos oder unter Überdruck, vorzugsweise mit Unterdruck, vorteilhaft bei 0,1 bis 250, insbesondere 0,1 bis 100 mbar, diskontinuierlich oder vorzugswei-

se kontinuierlich durchgeführt. In der Regel verwendet man keine zusätzlichen, unter den Reaktionsbedingungen inerten Lösungsmittel. Als Lösungsmittel kommen gegebenenfalls in Frage : aromatische, araliphatische, cycloaliphatische oder aliphatische Kohlenwasserstoffe, z. B. Toluol, Ethylbenzol, o-, m-, p-Xylol, Cyclohexan, Isooctan, Phenyldecan und entsprechende Gemische. Zweckmäßig verwendet man das Lösungsmittel in einer Menge von 400 bis 10 000 Gewichtsprozent, bezogen auf Ausgangsstoff II.

Als unter den Reaktionsbedingungen inerte Gase (Inertgas) werden zweckmäßig Alkane wie Methan, Ethan, Propan, 2,2-Dimethylpropan, Butan, Pentan, Isobutan ; bevorzugt Stickstoff, und/oder entsprechende Gemische verwendet. Es kommen zweckmäßig mindestens 0,001, vorzugsweise von 0,001 bis 1, insbesondere von 0,005 bis 0,5 Liter Inertgas je Liter Reaktionsraum und Stunde in Betracht.

Zweckmäßig kann man die Inertgasphase mittels Durchleiten von Inertgas durch den Reaktionsraum vor Zuführung des dampfförmigen Ausgangsstoffs oder zusammen mit ihm erzielen. Man setzt im allgemeinen in Abwesenheit von Sauerstoff oder sauerstoffhaltigen Gasgemischen, z. B. Luft, um, doch kann ein geringer Anteil, z. B. zwischen 0 und 10 Volumenprozent Sauerstoff, bezogen auf die Volumenmenge Inertgas, ohne wesentliche Verschlechterung der Ergebnisse anwesend sein.

Zweckmäßig verwendet man als Reaktionsräume rohrförmige Spaltreaktoren, die in einem weiten Bereich beliebige Reaktorquerschnitte besitzen können, vorteilhaft mit einem Durchmesser von 10 bis 100 Millimeter. Das Verhältnis von Länge zu Durchmesser der Reaktionsrohre (Spaltreaktoren) beträgt zweckmäßig 2 bis 1 000, vorzugsweise 5 bis 500 : 1. Die Spaltreaktoren können senkrecht oder waagrecht ausgerichtet sein und auch Zwischenlagen einnehmen.

Man führt die Spaltung vorteilhaft in Gegenwart von gasdurchlässigen, metallischen, unter den Reaktionsbedingungen thermisch stabilen Füllkörpern durch. Die Füllkörper können in einem weiten Bereich beliebige Formen besitzen. Als Füllkörper können z. B. Raschigringe, Intosringe, Prymringe, Pallringe, Berlsättel, Intaloxsättel, Torussättel, Interpackkörper, Stedmankörper, Schrägfilmblätter, Drahtnetzringe, Haltmeier-Rollen, Zwillingskörper, Wilsonspiralen oder Braunschweiger Wendeln verwendet werden. Bevorzugt sind metallische Perlen, Wolle, Ringe und/oder Späne. Anstelle von Metallen können auch Metallgemische, z. B. Metalllegierungen, verwendet werden. Als Metalle bzw. Metallgemische sind Kupfer, Titan, Chrom, Kobalt, Nickel, insbesondere Stahl, Messing, Aluminium und/oder Zink bevorzugt. Zweckmäßig verwendet man im kontinuierlichen Betrieb 10 bis 1 000, insbesondere 50 bis 300 Gramm Ausgangsstoff II je Liter Schüttvolumen und Stunde.

Die Reaktion kann wie folgt durchgeführt werden: Die Ausgangsstoffe II können in flüssiger oder fester Form, z. B. als Schmelze, als Pulver oder als Suspension oder Lösung in einem unter den Reaktionsbedingungen inerten Lösungsmittel, z. B. den vorgenannten, verwendet werden. Bevorzugt wird man sie jedoch unzersetzt verdampfen und gasförmig dem Spaltreaktor zuführen oder zweckmäßig in gasförmiger Form, gegebenenfalls im Gemisch mit Inertgas durch den Reaktionsraum, in dem eine Inertgassphäre, z. B. Stickstoffgasphase, herrscht, bei der Spalttemperatur leiten. Diese Arbeitsweise (Durchleiten zusammen mit einem Inertgas) oder Anlegen von Vakuum ist vorteilhaft, um die Spaltprodukte rasch aus dem Spaltreaktor auszuführen und gegebenenfalls eine effektive Trennung von Endstoff und gebildetem Alkohol zu erleichtern. Zweckmäßig richtet sich der in dem Reaktor einzustellende Druck nach den Siedepunkten der gebildeten Reaktionsprodukte Isocyanat I und Alkohol. Man wird bevorzugt Drücke verwenden, die es erlauben, den Endstoff rasch aus dem Reaktor auszuführen, die aber eine fraktionierende Kondensation noch ermöglichen. Die Belastung beträgt zweckmäßig 50 bis 1 000, insbesondere 100 bis 500 Gramm Ausgangsstoff II pro Liter Reaktionsraum und Stunde. Die Verweilzeit der Ausgangsstoffe II im Reaktionsraum beträgt zweckmäßig 0,01 bis 30, vorteilhaft 0,1 bis 10 Sekunden im kontinuierlichen oder diskontinuierlichen Betrieb. Nach der Reaktion wird das Reaktionsgemisch rasch, vorzugsweise binnen 1 bis 900, insbesondere 5 bis 600 Sekunden, auf eine Temperatur von weniger als 60 °C, zweckmäßig von 20 bis 59, vorzugsweise 25 bis 45 °C, insbesondere 30 bis 40 °C abgekühlt. Dann werden zweckmäßig der Endstoff in einer 1. Kondensationsstufe und dann der gebildete Alkohol in einer 2. Kondensationsstufe kondensiert und abgetrennt, wobei die Temperaturen der Kondensationsstufen je nach den physikalischen Daten von Isocyanat und Alkohol entsprechend dem Druck leicht ermittelt werden können.

Die Temperaturen der beiden Kondensatoren werden zweckmäßig so eingestellt, daß in der ersten Stufe vorwiegend das höhersiedende (z. B. Isocyanat) und in der zweiten Stufe tiefer siedende Produkt (z. B. der Alkohol) abgetrennt werden. Die in der ersten Kondensationsstufe erhaltenen 3-Dialkylamino-2,2-dialkylpropylisocyanate fallen üblicherweise in einer Reinheit von mehr als 80 % an (neben durch Rekombination rückgebildetem Ausgangsstoff II) und können durch Vakuumdestillation rein gewonnen werden.

In einer bevorzugten Ausführungsform des Verfahrens werden vor der Reaktion die verwendeten Füllkörper aktiviert, in der Regel mit Halogenwasserstoffsäuren, vorteilhaft Chlorwasserstoffsäure, zweckmäßig in der Gasphase, allein oder vorteilhaft im Gemisch mit Inergasen: Als Inertgase kommen die vorgenannten Gase, insbesondere Stickstoff, in Betracht. Zweckmäßig sind Verweilzeiten der Halogenwasserstoffe von 1 bis 120, vorteilhaft 10 bis 30 Minuten. Vorteilhaft beträgt in den Gemischen das Volumenverhältnis von Stickstoff zu gasförmigem Halogenwasserstoff 1 bis 100, insbesondere 4 bis 20. Die Aktivierung wird im allgemeinen bei einer Temperatur oberhalb 100 °C, zweckmäßig 200 bis 500, vorteilhaft 200 bis 400 °C, drucklos, unter Überdruck oder unter Unterdruck, diskontinuierlich oder vorteilhaft kontinuierlich durchgeführt. Nach der Aktivierung wird in der Regel durch einen Stickstoffstrom der restliche Anteil an Halogenwasserstoff entfernt und so die Inertgasphase für die folgende Spaltungsreaktion hergestellt.

Die nach dem Verfahren der Erfindung herstellbaren Isocyanate sind wertvolle Ausgangsstoffe für die Herstellung von Pflanzenschutzmitteln, Schädlingsbekämpfungsmitteln, Farbstoffen, Kunstharzen und Kunststoffen, Textilhydrophobierungsmitteln, Waschmitteln und Klebstoffen. Bezüglich der Verwendung wird auf vorgenannte Veröffentlichungen und Ullmanns Encyklopädie der technischen Chemie, 3. Auflage, Band 9, Seiten 11, 12, 404, und Band 17, Seite 204, verwiesen.

Beispiel 1

a) Aktivierung des Katalysators : Durch die Spaltapparatur, die aus einem Dünnschichtverdampfer, einem elektrisch beheizten Spaltreaktor aus Quarz, der mit 1 Liter $V_2A$-Maschendrahtringen (ca. 3 600 Maschen/$cm^2$) (gesamter Füllkörperbettquerschnitt 12,6 qcm) von 3 mm Durchmesser gefüllt ist, einer Kühleinrichtung und einer zweistufigen Kondensationseinrichtung bestand, wurde während 30 Minuten ein Strom aus Chlorwasserstoff und Stickstoff (Volumenverhältnis 1 : 20, Strömungsgeschwindigkeit 0,5 Liter HCl je Stunde) geleitet, wobei der Spaltreaktor bei 250 °C gehalten wurde. Danach spülte man die Apparatur mit Stickstoff chlorwasserstoffrei und evakuierte auf 10 mbar.

b) Spaltung : Nun verdampfte man innerhalb von 385 Minuten 310 g N-(3-Dimethylamino-2,2-dimethylpropyl)-carbamidsäuremethylester in dem auf 160 °C erhitzten Verdampfer, führte den Dampf durch den auf 530 °C erhitzten Spaltreaktor, kühlte das Reaktionsgemisch binnen 10 Sekunden im Kühler auf 39 °C ab und kondensierte es fraktionierend in den beiden nachfolgenden Kondensatoren. Die Verweilzeit im Reaktionsraum betrug 5 Sekunden. In dem ersten, bei 10 bis 12 °C betriebenen Kondensator erhielt man 187 g (96,7 % der Theorie) 3-Dimethylamino-2,2-dimethylpropylisocyanat vom Siedepunkt 40 °C/0,3 mbar neben 51 g Ausgangsstoff II. Weitere 26 g Ausgangsstoff wurden neben Methanol in der zweiten mit Trockeneis gekühlten Vorlage kondensiert. Der Umsatz betrug 75,2 %.

Beispiele 2 bis 6

Sie wurden analog Beispiel 1 ausgeführt, wobei jedoch die Ethylester der in der Tabelle genannten Carbamidsäuren zum Einsatz kamen.

| Beispiel Nr. | Menge an Ausstoff II in g | Ausgangsstoff II $R{-}NH{-}\underset{\underset{O}{\Vert}}{C}{-}OC_2H_5$ R | Endstoff I Siedepunkt | Ausbeute in % der Theorie |
|---|---|---|---|---|
| 2 | 102 | 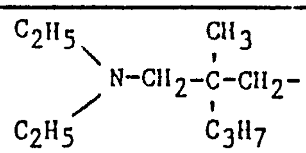 | 55°C/0,1 mbar | 92,0 |
| 3 | 105 | 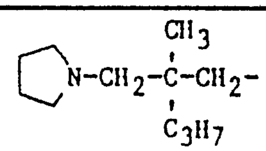 | 76°C/0,1 mbar | 94,5 |
| 4 | 111 | 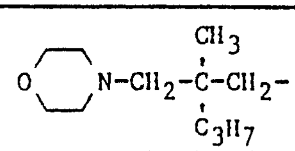 | 85°C/0,2 mbar | 93,5 |
| 5 | 102 | 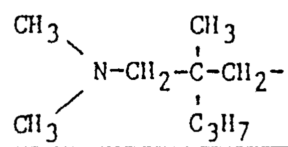 | 46°C/0,2 mbar | 89,7 |
| 6 | 40 | 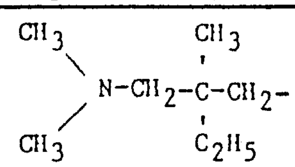 | 41°C/0,2 mbar | 78,1 |

Beispiel 7 (Vergleich)

35 Teile Oxalsäuremethylester-N-(3-dimethylamino-2-ethyl-2-methyl-propylamid wurden innerhalb 45 Minuten über eine Dosierschnecke von oben in einen 160 Volumenteile fassenden Rohrreaktor eingebracht, der mit Drahtringen gefüllt war und in dessen Inneren eine Temperatur von 600 °C und ein Druck von 19 mbar herrschten. Das untere Ende des Reaktors mündete in eine Vorlage, die mit Wasser gekühlt wurde ; in ihr wurde das gebildete Isocyanat kondensiert. Eine zweite, mit Trockeneis gekühlte Vorlage zur Kondensation des Methanols war nachgeschaltet.

Man erhielt bei einem Umsatz von (gaschromatographisch bestimmt) 71,2 % das 3-Dimethylamino-2-ethyl-2-methyl-propylisocyanat in einer Ausbeute von 57,3 % der Theorie vom Kp. 41 °C. Daneben wurden harzige Massen gebildet, von denen das Isocyanat nur schwer abgetrennt werden konnte.

**Patentansprüche**

1. Verfahren zur Herstellung von in 3-Stellung heterocyclischen oder 3-Dialkylamino-2,2-dialkyl-propyl-isocyanaten der Formel

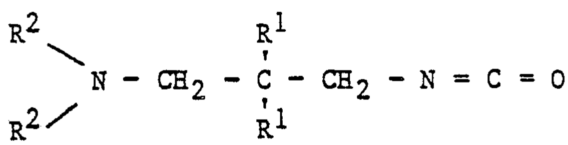
(I)

worin die einzelnen Reste $R^1$ und $R^2$ gleich oder verschieden sein können und jeweils einen aliphatischen Rest bedeuten, die Reste $R^2$ auch zusammen mit dem benachbarten Stickstoffatom Glieder eines heterocyclischen Restes bezeichnen, durch thermische Spaltung von Carbamidsäureestern, dadurch gekennzeichnet, daß man in 3-Stellung heterocyclische oder N-(3-Dialkylamino-2,2-dialkylpropyl)-carbamidsäureester der Formel

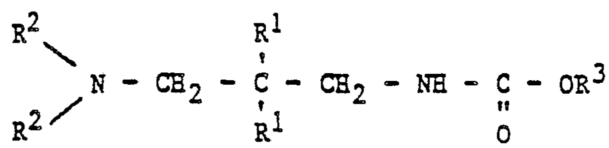
(II)

worin $R^1$ und $R^2$ die vorgenannte Bedeutung besitzen und $R^3$ einen aliphatischen, cycloaliphatischen, araliphatischen oder aromatischen Rest bezeichnet, in einer Inertgasphase bei einer Temperatur von mehr als 300 °C spaltet und das so gebildete Reaktionsgemisch auf weniger als 60 °C abkühlt.

2. Verfahren nach Anspruch 1, dadurch gekennzeichnet, daß man die Spaltung in einer Stickstoffphase in Gegenwart von gasdurchlässigen, metallischen, durch Halogenwasserstoff aktivierten Füllkörpern durchführt.

**Claims**

1. A process for the preparation of a 3-dialkylamino-2,2-dialkylpropyl isocyanate, or of a 2,2-dialkylpropyl isocyanate which is substituted in the 3-position by a heterocyclic radical, of the formula

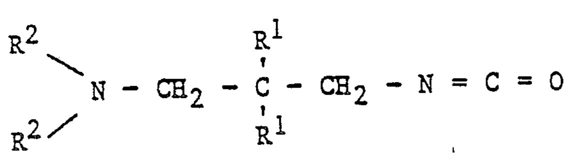
(I)

where the individual radicals $R^1$ and $R^2$ can be identical or different and are each an aliphatic radical, and the radicals $R^2$, together with the adjacent nitrogen atom, may furthermore be members of a heterocyclic radical, by thermal cleavage of a carbamate, wherein an N-(3-dialkyl-amino-2,2-dialkylpropyl)-carbamate, or an N-(2,2-dialkylpropyl)-carbamate which is substituted in the 3-position by a heterocyclic radical, of the formula

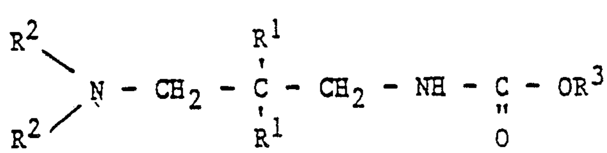
(II)

where $R^1$ and $R^2$ have the above meanings and $R^3$ is an aliphatic, cycloaliphatic, araliphatic or aromatic radical, is cleaved in an inert gas phase at above 300 °C, and the resulting reaction mixture is cooled to below 60 °C.

2. A process as claimed in claim 1, wherein the cleavage is carried out in a nitrogen phase in the presence of a metallic packing which is permeable to gas and has been activated by a hydrogen halide.

**Revendications**

1. Procédé de préparation d'isocyanates 3-hétérocyclo- ou 3-dialkylamino-2,2-dialkyl-propyliques de la formule

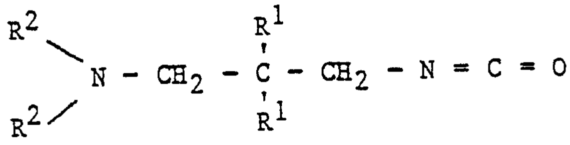

(I)

dans laquelle les différents substituants $R^1$ et $R^2$, qui peuvent être identiques ou différents, désignent chacun un groupe aliphatique, les substituants $R^2$ pouvant en outre constituer avec l'atome d'azote adjacent des maillons d'un groupe hétérocyclique, par clivage thermique d'esters de l'acide carbamique, caractérisé en ce que l'on soumet des 3-hétérocyclo- ou N-(3-dialkylamino-2,2-dialkyl-propyl)-carbamates de la formule

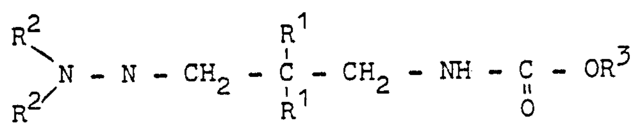

(II)

dans laquelle $R^1$ et $R^2$ possèdent la signification définie et $R^3$ désigne un groupe aliphatique, cycloaliphatique, araliphatique ou aromatique, à un clivage dans une atmosphère de gaz inerte et à une température supérieure à 300 °C, puis on refroidit le mélange réactionnel formé à une température inférieure à 60 °C.

2. Procédé suivant la revendication 1, caractérisé en ce que le clivage est réalisé en atmosphère d'azote en présence de corps de remplissage métalliques perméables aux gaz et activés par un hydrogène halogéné.